# EUROPÄISCHE PATENTSCHRIFT

(11) **EP 2 215 988 B1**
(45) Veröffentlichungstag und Bekanntmachung des Hinweises auf die Patenterteilung: **23.08.2017**
(21) Anmeldenummer: 10152860.2
(22) Anmeldetag: 08.02.2010
(51) Int. Cl.: A61C 1/00, A61B 18/22, A61C 1/18

(54) **Steuereinheit für ein Laserhandstück**
Control unit for a laser tool holder
Unité de commande pour pièce à main laser

(30) Priorität: 06.02.2009 DE 102009000685
(43) Veröffentlichungstag der Anmeldung: 11.08.2010
(60) Teilanmeldung: 17173168.0
(73) Patentinhaber: Sirona Dental Systems GmbH, 64625 Bensheim (DE)
(72) Erfinder: Bierbaum, Thomas, 64625, Bensheim (DE); Goisser, Siegfried, 64683, Einhausen (DE); Landgraf, Hermann, 64653, Lorsch (DE); Rein, Matthias, 64653, Lorsch (DE); Sutter, Ralf, 69469, Weinheim (DE); Jerger, Thomas, 72116, Mössingen (DE); Peuckert, Joachim, 74357, Bönnigheim (DE)
(74) Vertreter: Sommer, Peter

(56) Entgegenhaltungen:
- EP-A1- 0 882 432
- DE-A1- 1 942 987
- DE-T2- 69 327 187
- US-A- 5 976 124
- US-A- 6 102 926
- US-A1- 2005 154 379
- US-A1- 2007 191 823

## Beschreibung

### Technisches Gebiet

Die Erfindung betrifft eine Steuereinheit für ein Laserhandstück. Für die Bearbeitung von Zähnen oder Zahnfleisch in der Mundhöhle eines Patienten ist es bekannt, Laserlicht in einer Lasereinheit zu erzeugen und in den Patientenmund zu führen und auf die zu bearbeitende Stelle auszurichten.

### Stand der Technik

Dafür sind im wesentlichen zwei verschiedene Ausführungen bekannt. So ist eine bis zu 3 m lange Glasfaser am proximalen Ende mit einer Lasereinheit verbunden und das distale Ende wird zur Behandlung eingesetzt. Die Glasfaser wird nach jeder Anwendung aus hygienischen Gründen um ein definiertes Maß gekürzt. Sobald eine gewisse Mindestlänge des Abstands zwischen der Lasereinheit und dem Patient unterschritten wird, ist die restliche Glasfaser vollständig zu erneuen. Zur Führung des distale Endes der Glasfaser dient das Handstück, das auf die Glasfaser aufgeschoben wird und nach jedem Kürzen der Glasfaser an dieser entlang zurückgeschoben wird.

Weiterhin ist es bekannt, die Lasereinheit über einen Lichtleiter mit einem Handstück zu verbinden. Innerhalb des Handstückes erfolgt eine Lichtübertragung von dem Lichtleiter zu einer Glasfaser, welche zur Behandlung am Patienten eingesetzt wird. Diese Glasfasern werden heute zur Einmalverwendung angeboten und sind daher auswechselbar im Handstück befestigt.

In beiden Fällen erfolgt die Aktivierung der Lasereinheit und somit die Aktivierung des Laserstrahles über einen Fußschalter, wie in der Zahnarztpraxis üblich.

Aus der DE 693 27 187 T2 ist ein Laserhandstück bekannt, dem Laserstrahlung von einer externen Laserquelle zu einer wegwerfbaren oder verbrauchbaren optischen Faser aufweist. Diese Einweg- oder Wegwerffaser ist mit dem Handstück verbunden, wobei auf optische Komponenten und insbesondere Linsen zwischen einer in dem Handstück installierten optischen Faser zur Ausgabe von Laserstrahlung an das Handstück und einer austauschbaren optischen Ausgabefaser verzichtet werden kann, da eine zusätzliche Kühlung der Übergabestelle stattfindet.

Es sind weiterhin Ausführungen bekannt, bei denen ein Fingerschalter zur Aktivierung der Laserstrahlung dem Handstück zugeordnet sind.

Für die vorgesehene Wiederverwendung der Glasfaser lassen sich Verschmutzungen der Glasfaser, insbesondere im Bereich des für die Behandlung verwendeten und im Körperkontakt stehenden distalen Endes der Glasfaser auf einer letzten Länge von einigen Millimetern, hervorgerufen durch Karbonisierung von Körpergewebe nicht ohne Funktionsbeeinträchtigung beseitigen, sodass typischerweise die Glasfaser gekürzt wird. Die Ausführung mit einem auf der Glasfaser angeordneten verschiebbaren Handstück hat insbesondere in Verbindung mit einem elektrischen Fingerschalter den Nachteil, dass die Länge des Glasfaser von Anwendung zu Anwendung abnimmt, während die Länge der elektrischen Leitung typischerweise konstant bleibt. Eine Funkübertragung des Schaltsignals ist kompliziert, platzaufwändig und erfordern einen Energiespeicher. Besondere Bedeutung hat hierbei die Sicherheit, da eine mögliche Fehlaktivierung oder eine Nichtabschaltung des Lasers zu einer sehr erheblichen Gefährdung des Patienten, des Anwenders oder des Hilfspersonals führt.

Aus der DE 1 942 987 A1 ist eine Beleuchtungseinrichtung mit einer angeschlossenen Glasfaser bekannt, wobei die Glasfaser auf einer als Rückzugsvorrichtung ausgebildeten Trommel aufgewickelt und an eine in der Mitte der Trommel angeordnete Lichtquelle angekoppelt ist.

Die Aufgabe der Erfindung besteht darin, ein Steuergerät für ein Laserhandstück so auszubilden, dass der Lichtleiter für die Zuführung von Laserlicht zu dem Handstück bequem aufgewickelt und gehalten werden kann.

### Darstellung der Erfindung

Diese Aufgaben werden mit den unabhängigen Anspruch 1 gelöst, vorteilhafte Weiterbildungen sind in den Unteransprüchen angegeben.

Ein Gegenstand der Erfindung betrifft ein Steuergerät für ein Laserhandstück, umfassend einen mit dem Steuergerät verbunden Lichtleiter. Das Gehäuse weist einen umfangseitig umlaufenden Ringspalt auf, wobei der Ringspalt das Gehäuse in eine untere Gehäusehälfte und in eine obere Gehäusehälfte zumindest optisch teilt und wobei die obere Gehäusehälfte von vorne betrachtet seitlich über die untere Gehäusehälfte hervorsteht und die untere Gehäusehälfte von vorne betrachtet hinten seitlich über die obere Gehäusehälfte hervorsteht.

Damit ist die Zuleitung zum Handstück trotz einer Länge von ca. 2 Metern noch einfach handhabbar. Den unterschiedlichen räumlichen Bedingungen in der Zahnarztpraxis kann dadurch Rechnung getragen werden, dass der Anwender nicht die volle Länge der Zuleitung zum Handstück abwickeln muss.

Der Lichtleiter kann in einem mit dem Steuergerät verbundenen Handstückschlauch angeordnet sein. Dabei kann in dem Handstückschlauch auch zusätzlich eine Signalleitung oder eine Steuerleitung angeordnet sein, um eine Signalübertragung vom Laserhandstück an das Steuergerät zu ermöglichen.

Vorteilhafterweise kann die Breite des Ringspaltes mindestens dem einfachen Durchmesser und weniger als dem 2-fachen, vorzugsweise weniger als dem 1,2-fachen Durchmesser des Lichtleiters oder des Handstückschlauchs entsprechen, um ein definiertes Aufwickeln zu ermöglichen.

Vorzugsweise kann der Lichtleiter aus dem Gehäuseinneren knickfrei in den Grund des Ringspaltes geführt sein.

Gemäß einer Weiterbildung kann der Lichtleiter oder der Handstückschlauch elektrische und/oder optische Anschlüsse aufweisen und zu Wartungszwecke vom Steuergerät trennbar sein.

Weiterhin kann im Ringspalt eine Hemmung vorgesehen sein, die als mechanische Stopper, als Klettband oder als magnetischer Halter ausgebildet sein kann.

Am Gehäuse selbst kann eine Ablageeinrichtung für das Handstück vorgesehen sein, wobei insbesondere dann, wenn ein vorstehend beschriebenes Laserhandstück verwendet wird, eine Längenanpassung des Lichtleiters oder des Handstückschlauchs bei dem in der Ablage befindlichen Handstück möglich ist.

### Kurze Beschreibung der Zeichnung

In der Zeichnung ist Ausführungsbeispiel der Erfindung dargestellt. Es zeigt die:
- Fig. 1: Einen Laserhandstück in einer perspektivischen Ansicht, die
- Fig. 2: einen im Laserhandstück aus Fig. 1 innenliegenden und verschiebbaren Grundkörper, die
- Fig. 3: einen Schnitt durch den Grundkörper aus Fig. 2, die
- Fig. 4: eine Explosionszeichnung einer Griffhülse des Laserhandstücks aus Fig. 1, die
- Fig. 5: einen Längsschnitt durch das Laserhandstück aus Fig. 1, die
- Fig. 6: eine Seitenansicht eines Steuergeräts für das Laserhandstück aus Fig. 1, die
- Fig. 7: einen schematischen Schnitt durch das Steuergerät aus Fig. 6, die
- Fig. 8: einen auswechselbaren Glasfasereinsatz mit einer Aufbewahrungshülse, die
- Fig. 9A: eine Ausgestaltung der Kabelaufwicklung des Steuergeräts aus Fig. 6, die
- Fig. 9B: eine Kabelhemmung mit einer mechanischen Sperre und die
- Fig. 9C: eine magnetische Kabelhemmung.

### Ausführungsbeispiele der Erfindung

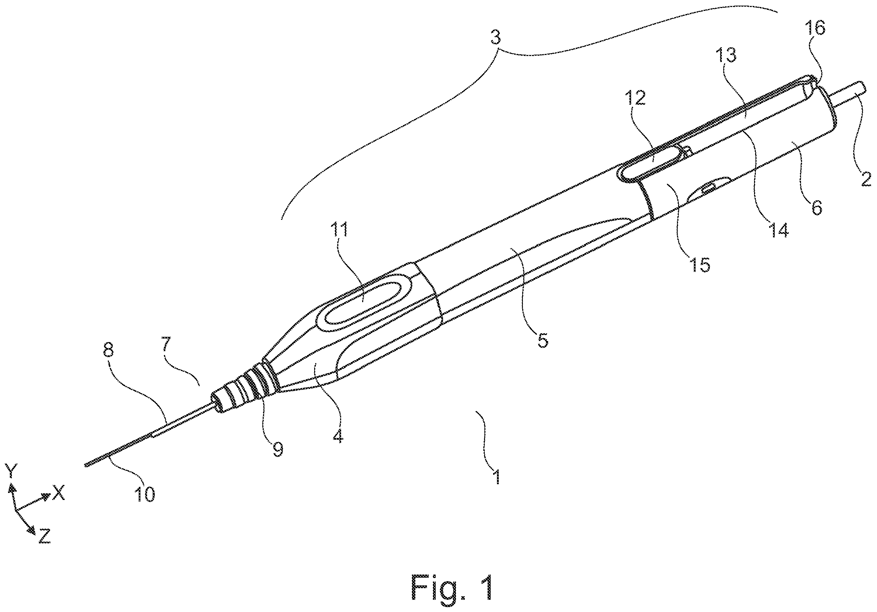

In Fig. 1 ist ein Laserhandstück für dentale Anwendungen in einer perspektivischen Darstellung gezeigt. Das Laserhandstück 1 weist einen Handstückschlauch 2 mit einem nicht dargestellten Lichtleiter und gegebenenfalls nicht dargestellten elektrischen Leitungen auf, wobei der Handstückschlauch 2 in einer Griffhülse 3 geführt ist. Die Griffhülse 3 kann mehrteilig ausgebildet sein und weist einen Fingerauflagebereich 4, einen Stützbereich 5 und einen Verstellbereich 6 auf, die in Längsrichtung gesehen hintereinanderliegend angeordnet sind. An dem dem Handstückschlauch 2 abgewandten Ende der Griffhülse 3 ist eine Spitze 7 mit einem Führungsröhrchen 8 und einem Anschlussteil 9 an die Griffhülse 3 vorgesehen. Aus dem Führungsröhrchen 8 der Spitze 7 tritt ein Applikationselement für Laserlicht in Form einer Glasfaser 10 aus, mittels derer das Laserlicht an den Ort der Behandlung geleitet wird. Derartige Glasfasern sind für die Behandlung von Weichgewebe und Hartgewebe bekannt. Nach jeder Benutzung des Laserhandstücks kann es erforderlich sein, die Glasfaser 10 zu kürzen, um in die Glasfaser 10 während der Bearbeitung eingebrannte Geweberückstände zu entfernen.

In Fig. 1 ist weiterhin zu erkennen, dass die Griffhülse 3 im Fingerauflagebereich 4 ein sich parallel zur Längsachse des Laserhandstücks erstreckendes Tastenfeld 11 aufweist, über welches auf einen darunter angeordnete, später erläuterte Tastschalter eingewirkt werden kann. Dieses Tastenfeld 11 ist zwischen 20 und 45 mm lang und ermöglicht die Betätigung des darunter liegenden Tastschalters unabhängig von dessen tatsächlicher Größe sowohl bei einer Frontzahnbehandlung, bei welcher das Laserhandstück weit vorne gegriffen wird, als auch bei einer Backenzahnbehandlung, bei das Laserhandstück weiter hinten gegriffen wird.

Weiterhin ist in Fig. 1 ein Verschiebeknopf 12 dargestellt, der in dem Verstellbereich 6 der Griffhülse 3 in einer Ausnehmung 13 angeordnet ist, wobei die Griffhülse eine Verschiebebahn 14 mit einer Führung für den Verschiebeknopf 12 aufweist. Der Verschiebeknopf 12 ist an einem in Inneren der Griffhülse 3 angeordneten hier nicht dargestellten Grundkörper befestigt, wobei dieser Grundkörper mittels des Verschiebeknopfs 12 entlang der Verschiebebahn 14 in der Griffhülse 3 längs verschiebbar ist. Das Zusammenwirken des Verschiebeknopfs 12 und der Führungsbahn 14 verhindert ein Verdrehen gegenüber der Griffhülse 3.

Der Verstellbereich 6 der Griffhülse 3 ist als separates Bauteil in Form einer Endhülse 15 ausgebildet und weist im Bereich der Ausnehmung 13 eine mit dieser verbundene endseitige Ausnehmung 16 auf, in welche der Handstückschlauch 2 einlegbar ist.

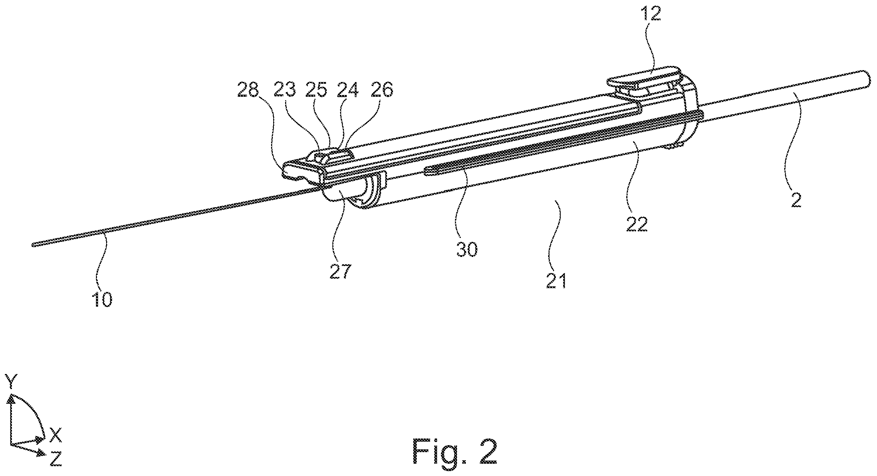

In Fig. 2 ist der vorstehend erwähnte, im Inneren der Griffhülse 3 aus Fig. 1 angeordnete Grundkörper 21 dargestellt, an dessen dem Handstückschlauch 2 zugewandten Ende der Verschiebeknopf 12 vorgesehen ist und an dessen gegenüberliegendem Ende die Glasfaser 10 angeordnet ist. Der Verschiebeknopf 12 ist auf der Oberseite des Gehäuses 22 des Grundkörpers 21 angeordnet, ebenso sind zwei Tastschalter 23, 24 vorgesehen, die in Längsrichtung gesehen versetzt zueinander angeordnet sind und die durch seitlich angeordnete Stege 25, 26 gegen ein unbeabsichtigtes Betätigen der Tastschalter 23, 24 gesichert sind. Die Höhe und der Abstand der Stege 25, 26 sind so bemessen, dass der in dem anzuwendenden Standard IEC 60601-1 beschriebene Normfinger eine Betätigung nicht erlaubt.

Die am Grundkörper 21 angeordnete Glasfaser 10 weist ein auch als Ferrule bezeichnetes Anschlussstück 27 auf, welches in dem Gehäuse 22 beispielsweise über ein Schraubgewinde lösbar befestigt ist, um im Falle des Verschleißes oder des Bruchs der Glasfaser 10 ein Auswechseln zu ermöglichen. Die Glasfaser 10 und das Anschlussstück 27 bilden zusammen den auswechselbaren Glasfasereinsatz. Die Oberseite des Gehäuses 22 überragt dieses Anschlussstück 27 nach vorne, so dass an der dem Anschlussstück 27 zugewandten Seite des Gehäusevorsprungs 28 ein Sensor vorgesehen sein kann, der das Vorhandensein des auswechselbaren Glasfasereinsatzes feststellt. Dies geschieht beispielsweise durch eine Erkennung von Licht im Bereich der Wellenlänge eines Führungslaserstrahls oder des Bearbeitungslaserstrahls, in dem beispielsweise die Intensität dieser Wellenlängen als Signal erfasst wird.

Die Führung des Grundkörpers in der Griffhülse kann auch über zusätzliche Nuten und Nocken erfolgen, wobei hier am Grundkörper ein längs verlaufender Nocken 30 erkennbar ist.

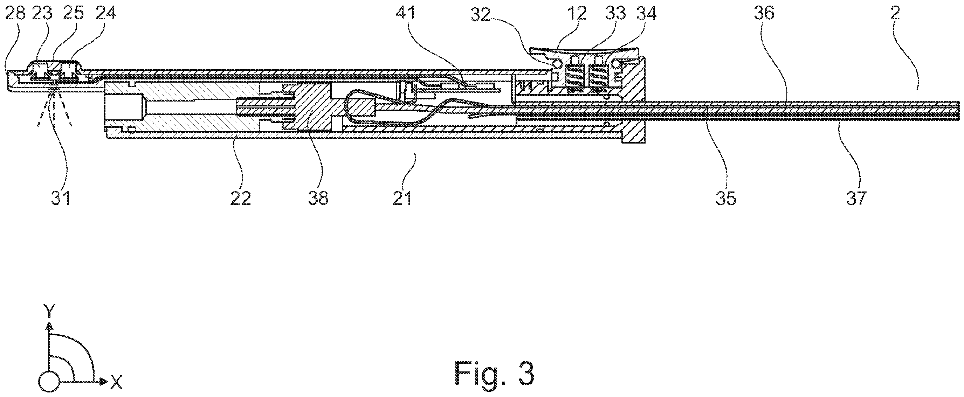

In Fig. 3 ist ein Schnitt längs durch den Grundkörper aus Fig. 2 dargestellt, wobei jedoch der auswechselbare Glasfasereinsatz weggelassen ist. Aus diesem Grund wird ein an der Unterseite des Vorsprungs 28 angeordneter Sensor 31 dann ein Fehlersignal liefern, wenn aus dem Gehäuse 22 des Grundkörpers 21 Laserstrahlen austreten.

Der Grundkörpers 21 ist mit dem an der Oberseite des Gehäuses 22 angeordneten Verschiebeknopf 12 versehen, der gegenüber dem Gehäuse 22 federnd eindrückbar gelagert ist und der einen umlaufenden O-Ring 32 aufweist, welcher mit der Verschiebebahn der Griffhülse aus Fig. 1 zum Zweck der Hemmung der Verschiebung zusammenwirkt. Durch Eindrücken des Verschiebeknopfs 12 wird der O-Ring 32 gegenüber der Verschiebebahn gelöst, sodass ein Verschieben begünstigt ist. Beim Loslassen des Verschiebeknopfs 12 wird dieser durch Federelemente 33, 34 gegen das Gehäuse 22 an die Verschiebebahn gedrückt.

Im Bereich des Verschiebeknopfs 12 mündet auch der Handstückschlauch 2 in das Gehäuse 22 ein und ist dort mittels einer Zugentlastung gehalten. Der Handstückschlauch 2 weist dabei zum einem einen Lichtleiter 35 auf, zum anderen Leitungen 36, wobei der Lichtleiter 35 und die Leitungen 36 von einer gemeinsamen Hülle 37 umschlossen sind. Bei den Leitungen 36 kann es sich um Signal- oder Steuerleitungen handeln, es können aber auch weitere Lichtleiter zur Signalübertragung vorhanden sein.

Im Inneren des Gehäuses 22 wird der Lichtleiter 35 bis zu einem optischen Lichteinkoppler 38 geführt und tritt aus dem optischen Lichteinkoppler 38, der auch optische Bauteile enthalten kann, wieder zum Applikationselement hinaus.

Das Gehäuse 22 weist darüber hinaus eine Leiterplatte 41 auf, an der die elektrischen Leitungen 36 kontaktiert sind und die mit den Tastschaltern 23, 24 und dem Sensor 31 elektrisch leitend verbunden ist. Dabei kann auch eine Auswerteeinheit auf der Leiterplatte 41 enthalten sein, um die von den Tastschaltern 23, 24 und dem Sensor 31 stammenden elektrischen Signale auszuwerten und die Bereitstellung des Laserlichts durch die Steuereinheit zu erlauben oder zu verhindern. Die Leiterplatte 41 kann auch lediglich zur Weiterleitung von Signalen an das Steuergerät dienen, wenn die Auswerteeinheit dort untergebracht ist. Die Signale können elektrisch oder optisch übertragen werden.

Das Gehäuse 22 des Grundkörpers ist hermetisch gegen die Umgebung abgedichtet.

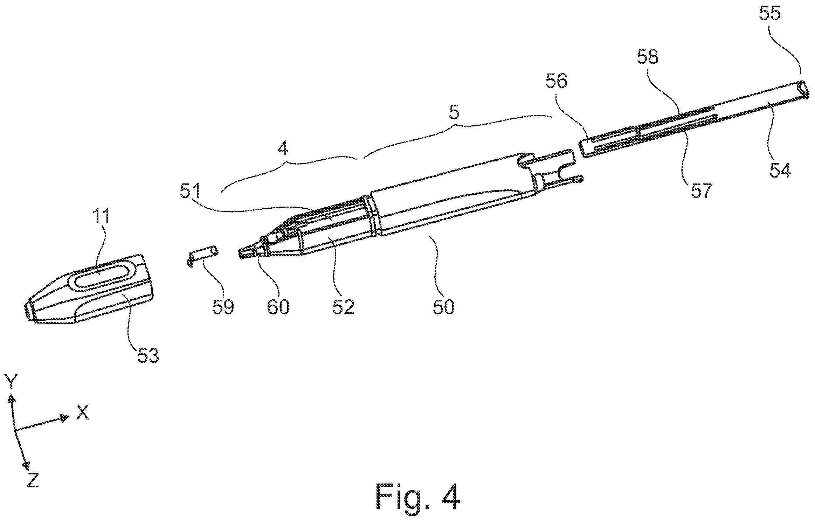

In Fig. 4 ist die Griffhülse 3 im Fingerauflagebereich 4 und Stützbereich 5 als Explosionszeichnung dargestellt, wobei im Fingerauflagebereich 4 an dem Hülsenkörper 50 eine Aussparung 51 vorgesehen ist, über welche die Tastschalter am nicht dargestellten Grundkörper aus Fig. 2 betätigbar sind. Dazu weist das vordere Ende 52 des Hülsenkörpers 50 einerseits einen abnehmbaren Überzug 53 mit einem verformbaren Tastenfeld 11 auf, andererseits ist ein Hebel 54 vorgesehen, der im Inneren des Hülsenkörpers 50 verläuft und die Ausnehmung 51 überdeckt. Der Hebel 54 ist nach dem Zusammenbau mit einem Drehgelenk 55 im Hülsenkörper 50 gelagert und das vordere Ende 56 liegt vollständig unter dem Tastenfeld 11. Damit der Hebel auf die Tastschalter des Grundkörpers aus Fig. 2 heruntergedrückt werden kann, sind Längsnuten 57, 58 vorgesehen, deren Breite so bemessen ist, dass die Sicherungsstege 25, 26 aus Fig. 2 dort eingreifen.

Um den Hebel 54 sicher in die abgehobene Endstellung zu bringen, ist weiterhin ein Federelement 59 vorgesehen, das im vorderen Teil 52 gehalten und mit dem Hebel 55 verbunden ist. Dieses Federelement 59 ist nicht zwingend erforderlich, sorgt aber für eine zusätzliche Sicherheit bei der Rückstellung des Hebels 54 über die Rückstellung durch die Tastschalter selbst hinaus.

Das Ende des Vorderteils 52 ist als Luer-Befestigung ausgebildet, damit ein Standardanschluss 60 zur Befestigung der nicht dargestellten auswechselbaren Spitze 7 aus Fig. 1 verwendet werden kann. Derartige Befestigungen sind im medizinischen Bereich Stand der Technik.

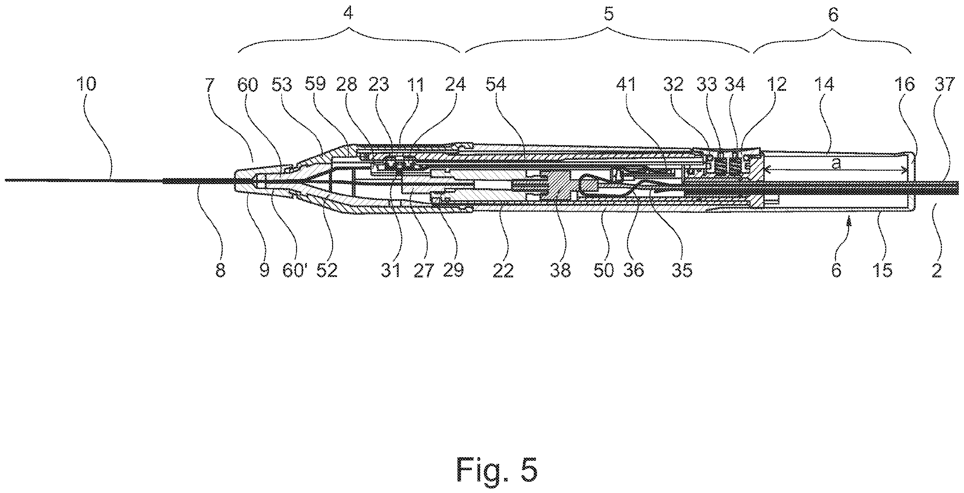

In Fig. 5 ist das zusammengebaute Laserhandstück aus den Fig. 1-4 im Längsschnitt dargestellt. Das Führungsröhrchen 8 der Spitze 7 ist fest im Anschlussteil 9 gehalten und der Anschlussteil 9 ist mit einem Luer-Anschluss 60' an das vordere Ende 52 des Hülsenkörpers 50 aus Fig. 4 ausgebildet, das ebenfalls einen entsprechenden Luer-Standardanschluss 60 aufweist.

Das Führungsröhrchen 8 kann bei eingebrachter Glasfaser mittels eines Biegewerkzeugs gebogen werden, ohne die eingebrachte Glasfaser zu beschädigen. Diese Biegung kann jedoch nicht rückgängig gemacht werden, außerdem ist es nicht möglich, die Glasfaser 10 durch die Spitze 7 zu schieben, wenn das Führungsröhrchen 8 abgebogen ist.

Die weiteren angegebenen Bauteile entsprechen denen aus den Fig. 1 - 4, wobei darauf hingewiesen wird, dass die Glasfaser 10 in eine maximal vorgeschobene Endstellung des Gehäuses 22 des Grundkörpers bezüglich des Hülsenkörpers 50 gebracht ist und damit um die maximale Länge über das Führungsröhrchen heraussteht. Beim Zurückschieben des Gehäuses 22 des Grundkörpers in der Endhülse 15 mittels des Verschiebeknopfs 12, der dazu eingedrückt wird, kann das Gehäuse 22 einen Verstellweg a zurücklegen. Aufgrund der Ausnehmung 16 am schlauchseitigen Ende des Endstücks 15 wird der Handstückschlauch 2 um diese Länge a aus dem Laserhandstück geschoben.

In dieser Schnittdarstellung ist auch der auf der Unterseite des Vorsprungs 28 angeordnete und auf den auswechselbaren Glasfasereinsatz, der aus der Glasfaser 10 und dem Anschlussstück 27 besteht, ausgerichtete Sensor 31 zur Detektion von Laserstrahlung erkennbar, der mit der Leiterplatte 41 elektrisch verbunden ist.

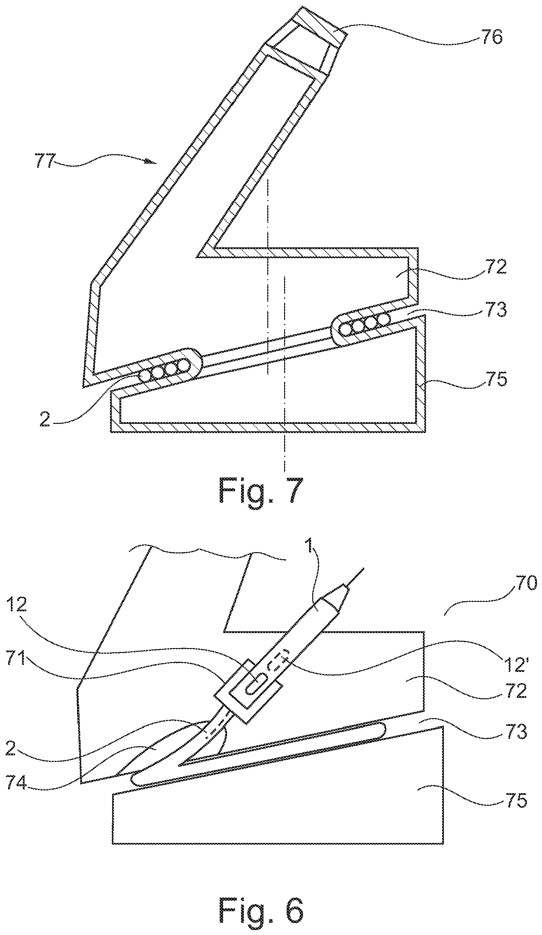

In Fig. 6 ist ein erste Ausführungsform eines erfindungsgemäßes Steuergerät zur Versorgung des vorstehend beschriebenen Laserhandstücks dargestellt, wobei das Laserhandstück 1 in einer Ablage 71 an einem Gehäuseteil 72 gehalten ist. Der Handstückschlauch 2 ist dabei in einen Ringspalt 73 eingelegt und verlässt diesen Ringspalt 73 erst kurz vor der Ablage 71. Um ein Abknicken zu vermeiden, kann das Gehäuseteil 72 eine Mulde 74 aufweisen. Mittels des Verschiebeknopfs 12 am Laserhandstück 1 kann die Schlauchlänge 2, die aus dem Ringspalt 73 herausgeführt ist, durch Verschieben in eine Position 12' gespannt werden, sodass der Handstückschlauch 2 auch beim Transport des Steuergeräts 70 nicht aus dem Ringspalt 73 fällt.

Bereits in Fig. 6 ist ersichtlich, dass der obere Gehäuseteil 72 des Steuergeräts 70 gegenüber dem unteren Gehäuseteil 75 versetzt angeordnet ist, wobei in der hier gewählten Seitenansicht auf die rechte Seite der Steuereinheit der obere Gehäuseteil 72 vorne, also in der Zeichnung auf der linken, dem Benutzer zugewandten Seite über das untere Gehäuseteil 75 hervorsteht, wohingegen auf der dem Benutzer abgewandten Seite, dass ist in der Zeichnung rechts, das untere Gehäuseteil 75 über das obere Gehäuseteil 72 hervorsteht. Dies wird beispielsweise dann erreicht, wenn ein Zylinder quer geschnitten wird und beide Zylinderteile zueinander einen Versatz aufweisen. Diese besondere Ausführung des Gehäuses des Steuergeräts hat den Vorteil, dass beim Aufwickeln des Handstückschlauchs 2 ein Auflegen des Handstückschlauchs 2 auf das untere Gehäuseteil 75 im vom Benutzer abgewandten Bereich des Steuergeräts möglich ist, wohingegen der Handstückschlauch an der dem Benutzer zugewandten Seite des Steuergeräts unter dem oberen Gehäuseteil hindurch geführt wird und beim Herumführen jeweils in die umlaufenden Ringspalt 73 eingelegt und damit aufgewickelt wird.

In dem in Fig. 7 schematisch dargestellten Schnitt wird deutlich, dass der Handstückschlauch 2 in dem Ringspalt 73 spiralförmig aufgewickelt ist, sodass jede Windung an der vorhergehenden anliegt. Bei festsitzender Aufwicklung ist der Handstückschlauch 2 daher immer in derselben Position in dem Ringspalt 73 abgelegt und das aus dem Ringspalt 73 herausragende Ende des in der Ablage 71 aus Fig. 5 abgelegten Handstücks ist immer gleich lang.

An dem Gehäuseteil 72 kann eine Anzeigevorrichtung angebracht sein, die hier in Form eines Pultes 77 ausgeführt ist, ebenso können nicht dargestellte Eingabemittel wie Tasten oder Drehregler vorgesehen sein, um eine nicht dargestellte, im Inneren des Gehäuses angeordnete Vorrichtung zur Bereitstellung von Laserstrahlen zu steuern. Weiterhin kann ein oberhalb des Pults 77 ein Griff 76 vorgesehen sein, um das Steuergerät einfach zu transportieren.

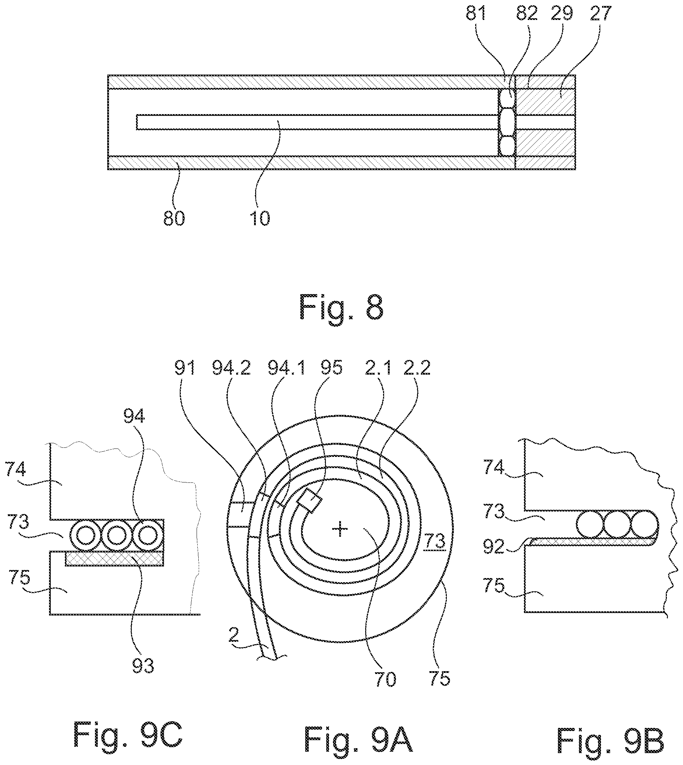

In Fig. 8 ist ein auswechselbarer Glasfasereinsatz dargestellt, wobei die Glasfaser 10 in dem Anschlussstück 27 befestigt ist und das Anschlussstück 27 einen mit einem Außengewinde 29 versehenen Halteabschnitt aufweist, zusammen auch bezeichnet als eine Wechselfieber. Anstelle eines Außengewindes 29 kann am Halteabschnitt auch ein Bajonettverschluss vorgesehen sein oder ein sonstiger Befestigungsmechanismus der durch Verdrehen des Anschlussstücks 27 eine Verbindung bewirkt.

Um das Anschlussstück 27 ohne Beschädigung der Glasfaser 10 befestigen zu können, etwa durch Einschrauben oder durch Verdrehen, ist eine Aufsteckhülse 80 vorgesehen, die sich um die Glasfaser 10 herum erstreckt und die einen Verbindungsbereich 81 zu einem Drehmomentübertragungsbereich 82 am Anschlussstück 27. Bei dem Drehmomentübertragungsbereich 82 kann es sich etwa um eine Art Sechskant-Mutter für einen Steckschlüssel handeln, noch besser handelt es sich aber um eine Verbindung mit lediglich punktförmigen Auflageflächen, etwa einer einseitigen Abflachung oder um einem z. B. senkrecht zur Längsachse eingebrachten Stift. Auch ein zum Festschrauben auf Reibschluss ausgelegter rotationssymmetrischer Drehmomentübertragungsbereich ist anwendbar. Es ist die Lösung zu bevorzugen, die beim Festschrauben die Möglichkeit bietet, das Drehmoment beim Festschrauben einfach zu begrenzen.

Die Aufsteckhülse 80 mit gleichzeitiger Werkzeugausformung dient einerseits als Transportschutz für die Glasfaser, als Werkzeug zum Einschrauben des auswechselbaren Glasfasereinsatzes in den Grundkörper 21 aus Fig. 2 und als Behältnis für die Glasfaser während der Sterilisation.

In Fig. 9A - 9C sind verschiedene Möglichkeiten einer Hemmung gezeigt, die eine windungsweise Abwicklung des Handstückschlauchs 2 ermöglichen. In Fig. 9A sind zunächst zwei Windungen 2.1, 2.2 des im Gehäuse vorgesehenen Ringspalts 73 des Steuergeräts 70 aufgewickelten Handstückschlauchs 2 dargestellt, wobei ein Halteelement 91 in einer Stellung bei neun Uhr vorgesehen ist. Durch dieses Halteelement 91 wird verhindert, dass sich die aufgewickelten Windungen 2.1, 2.2 des Handstückschlauchs 2 unbeabsichtigt lockern. Die kann dadurch erreicht werden, dass im Ringspalt 73 zwischen dem oberen und dem unteren Gehäuseteil 74, 75 als Halteelement 91 ein Silikonwulst oder eine elastische Auflage 92 vorgesehen ist. Alternativ dazu kann, wie in Fig. 9C dargestellt, ein unterhalb des Ringspalts 73 im unteren Gehäuseteil 75 angeordneter Magnet 93 als Halteelement vorgesehen sein, der mit einer magnetischen Manschette 94 am Handstückschlauch 2, dargestellt in Fig. 9A als Schlauchmanschette 94.1 und 94.2, zusammenwirkt.

Anstelle des Magnets und der Hülsen kann auch eine Klettverbindung vorgesehen sein.

Der Lichtleiter oder der Handstückschlauch wird vom Innern des Steuergeräts 70 knickfrei in den Ringraum 73 geführt und ist im Innern über einen Anschluss 95 mit dem Steuergerät 70 verbunden. Über den Anschluss 95 kann der Lichtleiter 35 oder der Handstückschlauch 2 zu Reparatur- oder Wartungszwecken vom Steuergerät getrennt werden.

### Bezugszeichenliste

- 1: Laserhandstück
- 2: Handstückschlauch
- 2.1: Windung
- 2.2: Windung
- 3: Griffhülse
- 4: Fingerauflagebereich
- 5: Stützbereich
- 6: Verstellbereich
- 7: Spitze
- 8: Führungsröhrchen
- 9: Anschlussteil
- 10: Glasfaser - Applikationselement
- 11: Tastenfeld
- 12, 12': Verschiebeknopf
- 13: Ausnehmung
- 14: Verschiebebahn
- 15: Endhülse
- 16: Ausnehmung
- 21: Grundkörper
- 22: Gehäuse
- 23: Tastschalter
- 24: Tastschalter
- 25: Steg/Sicherungssteg
- 26: Steg/Sicherungssteg
- 27: Anschlussstück
- 28: Vorsprung
- 29: Außengewinde / Halteabschnitt
- 30: Nocken
- 31: Sensor
- 32: O-Ring
- 33: Federelement
- 34: Federelement
- 35: Lichtleiter
- 36: Leitung / Signalleitung / Steuerleitung
- 37: Hülle
- 38: Lichteinkoppler
- 41: Leiterplatte / Auswerteeinheit
- 50: Hülsenkörper
- 51: Aussparung/Ausnehmung
- 52: vorderes Ende/vorderer Teil
- 53: Überzug
- 54: Hebel
- 55: Drehgelenk/Hebel
- 56: vorderes Ende
- 57: Längsnut
- 58: Längsnut
- 59: Federelement
- 60: Standardanschluss Luer am Gehäuse
- 60': Luer-Anschluss an der Spitze
- 70: Steuergerät
- 71: Ablage
- 72: Gehäuseteil
- 73: Ringspalt
- 74: Mulde
- 75: Unterteil/Gehäuseteil
- 76: Griff
- 77: Pult
- 80: Aufsteckhülse
- 81: Verbindungsbereich
- 82: Drehmomentübertragungsbereich
- 83: Halteabschnitt
- 91: Halteelement
- 92: Auflage
- 93: Magnet
- 94: Manschette
- 94.1: Schlauchmanschette
- 94.2: Schlauchmanschette
- 95: Anschluss für Lichtleiter / Handstückschlauch
- a: Verschiebeweg

## Patentansprüche

1. Steuergerät für ein Laserhandstück (1), umfassend einen mit dem Steuergerät (70) verbunden Lichtleiter (35), wobei das Gehäuse (74, 75) einen umfangseitig umlaufenden Ringspalt (73) aufweist, wobei der Ringspalt (73) das Gehäuse in eine untere Gehäusehälfte und in eine obere Gehäusehälfte (74, 75) zumindest optisch teilt und, **dadurch gekennzeichnet, dass** die obere Gehäusehälfte (74) von vorne betrachtet seitlich über die untere Gehäusehälfte (75) hervorsteht und die untere Gehäusehälfte (75) von vorne betrachtet hinten seitlich über die obere Gehäusehälfte (74) hervorsteht.

2. Steuergerät nach Anspruch 1, **dadurch gekennzeichnet, dass** der Lichtleiter (35) in dem Ringspalt (73) aus dem Gehäuseinneren austritt und im Ringspalt (73) aufwickelbar geführt ist.

3. Steuergerät nach Anspruch 1 oder 2, **dadurch gekennzeichnet, dass** die Breite des Ringspalts (73) mindestens dem einfachen Durchmesser und weniger als dem 2-fachen Durchmesser des Lichtleiters (35) oder des Handstückschlauchs (2) entspricht.

4. Steuergerät nach einem der Ansprüche 1 bis 3, **dadurch gekennzeichnet, dass** der Lichtleiter (35) oder der Handstückschlauch (2) aus dem Gehäuseinneren knickfrei in den Grund des Ringspaltes (73) geführt ist.

5. Steuergerät nach einem der Ansprüche 1 bis 4, **dadurch gekennzeichnet, dass** der Lichtleiter (35) oder der Handstückschlauch (2) einen elektrischen und/oder optischen Anschluss (95) aufweist und zu Wartungszwecke vom Steuergerät (70)trennbar ist.

6. Steuergerät nach einem der Ansprüche 1 bis 5, **dadurch gekennzeichnet, dass** im Ringspalt (73) eine Hemmung (92; 93) vorgesehen ist, die insbesondere als mechanische Stopper (92), als Klettverbindung oder als magnetischer Halter (93) ausgebildet ist.

## Claims

1. Control unit for a laser tool holder (1), comprising an optical fiber (35) connected to the control unit (70), wherein the housing (74, 75) has a annular gap (73) traveling around the circumference, wherein the annular gap (73), at least optically, divides the housing into a lower housing half and an upper housing half (74, 75), and **characterized in that** the upper housing half (74) projects laterally beyond the lower housing half (75) if viewed from the front, and the lower housing half (75) projects laterally beyond the upper housing half (74) if viewed from the rear.

2. Control unit according to claim 1, **characterized in that** the optical fiber (35) exits into the annular gap (73) from the inside of the housing and is directed into the annular gap (73) so that it can be coiled.

3. Control unit according to claim 1 or 2, **characterized in that** the width of the annular gap (73) corresponds to at least the simple diameter and less than twice the diameter of the optical fiber (35) or of the tool holder tube (2).

4. Control unit according to one of claims 1 through 3, **characterized in that** the optical fiber (35) or the tool holder tube (2) is directed, without any kinks, from the inside of the housing into the base of the annular gap (73).

5. Control unit according to one of claims 1 through 4, **characterized in that** the optical fiber (35) or the tool holder tube (2) has an electrical and/or optical connection (95) and can be disconnected from the control unit (70) for maintenance purposes.

6. Control unit according to one of claims 1 through 5, **characterized in that** a restraint (92; 93) is provided in the annular gap (73), which restraint is especially designed as a mechanical stopper (92), a hook-and-loop fastener, or a magnetic retainer (93).

## Revendications

1. Appareil de commande pour une pièce à main laser (1), comprenant un conducteur de lumière (35) relié à l'appareil de commande (70), dans lequel le boîtier (74, 75) présente une fente annulaire (73) qui s'étend autour de sa périphérie, dans lequel la fente annulaire (73) divise au moins visuellement le boîtier en une moitié inférieure et une moitié supérieure (74, 75) de boîtier, et **caractérisé en ce que** la moitié supérieure (74) de boîtier, vue de face, dépasse latéralement de la moitié inférieure (75) de boîtier et la moitié inférieure (75) de boîtier, vue de face, dépasse à l'arrière, latéralement, de la moitié supérieure (74) de boîtier.

2. Appareil de commande selon la revendication 1, **caractérisé en ce que** le conducteur de lumière (35) sort de l'intérieur du boîtier dans la fente annulaire (73) et est guidé de manière à pouvoir être enroulé dans la fente annulaire (73).

3. Appareil de commande selon la revendication 1 ou 2, **caractérisé en ce que** la largeur de la fente annulaire (73) correspond au moins au diamètre simple et à moins de 2 fois le diamètre du conducteur de lumière (35) ou du flexible (2) de la pièce à main.

4. Appareil de commande selon l'une quelconque des revendications 1 à 3, **caractérisé en ce que** le conducteur de lumière (35) ou le flexible (2) de la pièce à main est guidé sans coude dans le fond de la fente annulaire (73).

5. Appareil de commande selon l'une quelconque des revendications 1 à 4, **caractérisé en ce que** le conducteur de lumière (35) ou le flexible (2) de la pièce à main présente un raccord (95) électrique et/ou optique et peut être séparé de l'appareil de commande (70) à des fins d'entretien.

6. Appareil de commande selon l'une quelconque des revendications 1 à 5, **caractérisé en ce qu'**un blocage (92 ; 93) est disposé dans la fente annulaire (73), qui est en particulier réalisé sous forme d'arrêt mécanique (92), de liaison agrippante ou de fixation magnétique (93).
